# EUROPEAN PATENT APPLICATION

(11) **EP 4 610 993 A1**
(43) Date of publication of application: **03.09.2025**
(21) Application number: 25154644.6
(22) Date of filing: 29.01.2025
(51) Int. Cl.: G16H 10/60, G16H 80/00

(54) **COMPUTER-IMPLEMENTED METHOD AND SYSTEM FOR SHARING DATA BETWEEN CUSTOMER INSTITUTIONS**

(30) Priority: 29.02.2024 IN 202411014958
(71) Applicant: Siemens Healthineers AG, 91301 Forchheim (DE)
(72) Inventor: Anandaram, Ashok Shidlaghatta, 560079 Bangalore (IN); Manuel, Sujith, 91052 Erlangen (DE); Palleda Jagadish, Vilasa, 572201 Tiptur (IN)
(74) Representative: Siemens Healthineers Patent Attorneys

(57) **Abstract**

The present invention relates to a method and system for sharing data between customer institutions participating in a digital health platform. The method comprising: providing at least one data compartment adapted to store data of an application of a source institution; selecting a data compartment by a user of the source institution to generate a data compartment sharing request for sharing the selected data compartment with a specified target institution; notifying a user of the target institution about the data compartment sharing request of the user of the source institution; approving or rejecting the data compartment sharing request of the user of the source institution by a user of the target institution; notifying the user of the source institution about the approval or rejection of the data compartment sharing request by the user of the target institution; and providing access to the data stored in the selected shared data compartment of the source institution to the target institution if the data compartment sharing request is approved by the user of the target institution.

## Description

The present invention relates to a computer-implemented method and system for sharing data between customer institutions participating in a digital health platform.

The present invention may be applied, for example, in the environment of so-called data silos. A data silo refers to a situation in which data is stored or managed in isolation, separate from the rest of an organization's data. This isolation may occur for various reasons, such as different departments using different systems that do not communicate with each other, or organizational structures that hinder the sharing of information.

When data is stored in data silos, it becomes difficult for different parts of an organization to access and share information seamlessly. This lack of integration can lead to inefficiencies, duplication of efforts, and hindered collaboration among different teams or departments. It can also impede the organization's ability to derive meaningful insights from its data, as a holistic view of information is often necessary for comprehensive analysis.

Customers in different institutions such as hospital institutions or other health related institutions work mostly with patient data stored in their own data silos. However, when there is a need for customers of an institution to get additional diagnostic opinions from other customers or experts outside their local institution, this is not easily possible or almost impossible with way too many manual steps involving several approval steps considering the data privacy and security aspects on individual subject or patient level.

Further in case that institutions use vendor specific advanced tools for diagnosis and if that can only be used locally within the respective institution, there is no way for customers of the institution to provide diagnosis for the data which is stored in a data base of another institution.

There exist conventional sharing tools for sharing DICOM images via cloud-based web clients, but this still does makes this image data locally available so that it can be processed and interpreted in local advanced diagnostic tools.

Against this technical background, it is an object of the present invention to facilitate the collaboration between customers of different institutions when sharing and processing health related data.

According to the present invention, this object is solved by a computer-implemented method comprising the features of claim 1 and/or by a system comprising the features of claim 12.

According to a first aspect, the present invention provides a computer-implemented method for sharing data between customer institutions participating in a digital health platform. The method comprises the steps: providing at least one data compartment adapted to store data of an application of a source institution; receiving a data compartment sharing request issued by a user of the source institution via a terminal device of the respective user, wherein the data compartment sharing request for sharing a selected data compartment selected by the user with a specified target institution; notifying a terminal device of a user of the target institution about the data compartment sharing request; receiving an approval or rejection of the data compartment sharing request issued by the user of the target institution via the terminal device of the user of the target institution; notifying the terminal device of the user of the source institution about the approval or rejection of the data compartment sharing request; and in the case of approval, providing access to the data stored in the selected shared data compartment of the source institution to the terminal device of a user of the target institution.

Whereas conventional solutions only work locally within a single institution premise the computer-implemented invention according to the present invention spans across different customer institutions which can spread across different geographical locations.

The method according to the present invention makes it significant easier for a customer or a user of an institution to grant access to his/her data sets to a single or a group of other institutions. A customer or user of an institution can thus save time and work related to manual posting of data sets across different sites. A customer or user of an institution can reduce daily routine tasks time by reducing the amount of time spent by him for sharing data sets.

Moreover, in emergency situations the method according to the present invention can provide real-time access to patient data. For example, an ambulance acquiring images can make the acquired image data available to an institution on the fly.

A data compartment refers to a discrete storage area which can be established for a specific customer institution. A data compartment can serve multiple purposes such as an interim cloud-based storage for a specific customer facing application or as a data collection space for the customer for potential research scenarios.

A terminal device refers to an endpoint or device that communicates with a network and typically represents the point where a user or subsystem interacts with the network. Terminal devices comprise end-user devices that are adapted to provide or consume data, such as computers, smartphones, tablets, printers, or any other equipment that connects to a network.

In the health-related platform, various institutions can play a role in providing healthcare services, conducting research, and ensuring public health.

Key institutions which can be found in a health-related system may comprise at least one of the following institutions:
Hospitals and clinics:
   Provide primary, secondary, and tertiary healthcare services, including emergency care, surgeries, and specialized treatments.
Primary Care Centers:
   Offer basic and preventive healthcare services, often serving as the first point of contact for individuals seeking medical assistance.
Pharmacies:
   Dispense medications and provide information on their proper use.
Health Insurance Companies:
   Offer health insurance plans to individuals and organizations, covering medical expenses and providing financial support for healthcare services.
Public Health Agencies:
   Responsible for monitoring and promoting public health, managing disease outbreaks, and implementing preventive measures.
Research Institutions:
   Conduct medical and health-related research to advance scientific knowledge, develop new treatments, and improve healthcare practices. Examples include the National Institutes of Health (NIH).
Medical Schools and Universities:
   Provide education and training for healthcare professionals, including doctors, nurses, pharmacists, and researchers.
Government Health Departments:
   Oversee public health policies, regulations, and initiatives, and may be responsible for managing healthcare delivery systems. Examples include the Ministry of Health in various countries.
Diagnostic Laboratories:
   Perform medical tests and analyses, playing a crucial role in disease diagnosis and monitoring.
Rehabilitation Centers:
   Provide services for individuals recovering from illnesses, surgeries, or injuries, offering rehabilitation programs and therapies.
Mental Health Institutions:
   Focus on the diagnosis and treatment of mental health conditions, including psychiatric hospitals and counseling centers.
Long-Term Care Facilities:
   Offer care and support for individuals with chronic illnesses or disabilities who require extended periods of assistance, such as nursing homes and assisted living facilities.

These afore-mentioned institutions may collectively contribute to the overall functioning of a comprehensive health system, addressing various aspects of healthcare delivery, research, and public health. The specific institutions present may vary based on the healthcare system and structure within different countries.

The computer-implemented method of the present invention can be performed by a program executed on a process of a terminal device of a user of an institution INST participating in the digital health platform DHP implementing the system according to the present invention.

The invention provides according to a further aspect a terminal device comprising a processor adapted to execute a computer-implemented method according to the first aspect of the present invention.

The present invention provides according to a further aspect a computer program product having a stored program code adapted to execute a computer-implemented method for sharing data between customer institutions participating in a digital health platform, the steps: providing at least one data compartment adapted to store data of an application of a source institution; receiving a data compartment sharing request issued by a user of the source institution via a terminal device of the respective user, wherein the data compartment sharing request for sharing a selected data compartment selected by the user with a specified target institution; notifying a terminal device of a user of the target institution about the data compartment sharing request; receiving an approval or rejection of the data compartment sharing request issued by the user of the target institution via the terminal device of the user of the target institution; notifying the terminal device of the user of the source institution about the approval or rejection of the data compartment sharing request; and in the case of approval, providing access to the data stored in the selected shared data compartment of the source institution to the terminal device of a user of the target institution.

The program code can be stored on any kind of machine-readable data storage medium, such as a CD, DVD, USB stick or the like.

The invention provides according to a further aspect a system for sharing data between customer institutions participating in a digital health platform. The system comprises a compartment storage adapted to store data in logically isolated data compartments established for customer institutions. The system further comprises a cloud based distributed system adapted to receive a data compartment sharing request for sharing a selected data compartment with a specified target institution in response to a selection of a data compartment of a source institution made by a user of the source institution. The cloud based distributed system is further adapted to provide automatically access to the data stored in the selected data compartment of the source institution to the target institution if the data compartment sharing request has been approved by a user of the target institution.

Advantageous configurations and developments emerge from the further dependent claims and from the description with reference to the figures of the drawings.

In a possible embodiment of the method according to the present invention, the target institution is specified by the user of the source institution by indicating an institution name, an identifier, a serial number and/or an address of the target institution. This avoids misunderstandings or errors in the selection of the target institutions. The user of the source institution may select one or more suitable target institutions participating in the health related platform from an institution list prompted on a display of a user interface of a terminal device of the user.

In a possible embodiment, the data compartment sharing request generated by the terminal of the user of the source institution specifies at least one owner application or at least one third party application available on the target institution to have access to the data stored in the selected data compartment of the source institution. This facilitated collaboration between customers using the same or different applications. The application can be executed in the target institution outside the specified terminal.

In a possible embodiment, the data compartment sharing request specifies a time frame indicating a time period where the data sharing is active. This way, a customer or user of an institution can offer access to data sets stored in a data compartment of his institution for a limited time-period to other customers at other institutions. The length of the time-frame for data access can be determined and adjusted by the user sharing the data sets. This enhances the flexibility of the data sharing process for different scenarios and use cases.

In a possible embodiment, the data compartment sharing request specifies a level of access to the data stored in the selected data compartment of the source institution. This allows a customer or user of an institution to control the extent of access to shared data sets.

In a possible embodiment, the level of access specified in the compartment sharing request comprises allowed data operations including reading data, writing data, deleting data and execution of operations on data.

In a possible embodiment, the data stored in the data compartment comprises associated data properties including an indication of a purpose of data stored in the respective data compartment and data retention policies indicating a level of retention and/or a duration of data stored in the respective data compartment and a privacy level used for data minimization operation. This provides additional information for other users processing shared data.

In a possible embodiment, the user of the target institution is enabled by an authorization mechanism to configure the shared data compartment of the source institution. This can facilitate and accelerate data evaluation of data stored in the shared data compartment.

In a possible embodiment, the affected third party applications are notified automatically about the data compartments shared with them. This way, an application can become aware of all data compartments stored in different locations of the distributed system offering data access to the respective application.

In a possible embodiment, sharing of the shared data compartment of the source institution is terminated automatically in response to a compartment un-sharing request triggered by a user of the source institution or triggered by an event. This event can comprise the expiration of the time-period where the data sharing is active as specified in the compartment sharing request or other observed or detected events. This increases the flexibility of the method for adaption to a wide range of different use cases.

In a possible embodiment, a user of the target institution and affected third party applications are notified automatically about a termination of the sharing of the data compartment of the source institution.

In a possible embodiment of the cloud based distributed system, the system is further adapted to notify a user of the target institution about the received data compartment sharing request issued by the terminal device of the user of the source institution. The system is further adapted to notify the terminal of the user of the source institution about the approval or rejection of the data compartment sharing request by the user of the target institution. In this way, the customers on both sides become immediately aware whether sharing of data can be performed.

In a possible embodiment, the system further comprises a network connecting a plurality of customer institutions and adapted to transport messages and notifications between the institutions according to a predefined communication protocol. This facilitates communication between customers and data sharing between different institutions.

In a possible embodiment, the customer institutions form application entities in a DICOM (Digital Imaging and Communications in Medicine) network, wherein each data compartment exposes unique application entity titles (AETs).

In a possible embodiment of the cloud based distributed system the system comprises a DICOM server comprising for its identification and communication an IP-number, a port number and one or more application entity titles (AETs) belonging to a single or multiple data compartments.

In a possible embodiment, the system is inter-operable with other systems. These systems can be connected to the system of the present invention via data and/or communication interfaces. This offers a wide range of use cases for the system according to the present invention. The other connected inter-operable systems can include at least one of:
- imaging modalities, in particular medical imaging devices including X-ray machines, CT scanners, MRI machines adapted to generate medical images;
- modality worklist servers adapted to manage worklists for imaging modalities and/or providing information about scheduled procedures;
- image acquisition devices for acquiring medical images from imaging modalities and sending them to a Picture Archiving and Communication System (PACS) adapted to store, retrieve, and distribute medical images;
- viewer components adapted to retrieve medical images from the Picture Archiving and Communication System (PACS) and used by healthcare professionals to view and interpret the retrieved medical images;
- Radiology Information Systems (RIS) adapted to manage radiological information, including patient scheduling, reporting, and other administrative functions;
- print servers used for printing medical images to hardcopy films or other output devices.

Where appropriate, the above-mentioned embodiments can be combined with each other as desired, as far as this is reasonable. Further possible configurations and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- Fig. 1: shows a flow chart of a possible exemplary embodiment of a computer-implemented method according to a first aspect of the present invention;
- Fig. 2: shows a schematic diagram of an exemplary embodiment of a system according to further aspect of the present invention;
- Fig. 3: shows schematically a customer tenant comprising several data compartments which can be shared using the computer-implemented method according to the present invention;
- Fig. 4: shows schematically the offering to share a data compartment by a user of a source institution to a user of a target institution using the computer- implemented method according to the present invention;
- Fig. 5: shows schematically the approval or rejection of an offered data compartment sharing by a user of a target institution using the computer-implemented method according to the present invention;
- Fig. 6: shows schematically the configuration of shared data compartments using the computer-implemented method according to the present invention;
- Fig. 7: shows schematically the sharing of data compartments by different institutions using the computer-implemented method according to the present invention;
- Fig. 8: shows schematically the accessibility of data via third party application using the computer-implemented method according to the present invention; and
- Fig. 9: shows schematically the removal of an existing data compartment sharing using the computer-implemented method according to the present invention.

The appended drawings areintended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

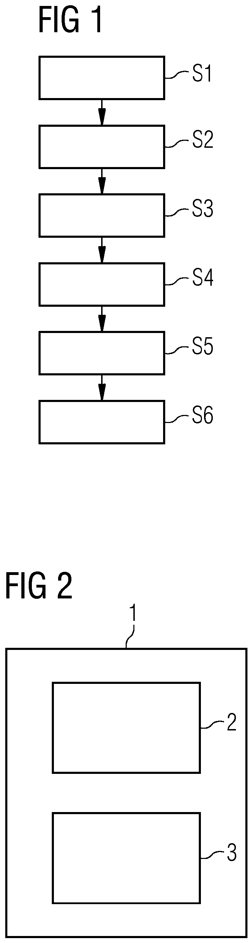

Fig. 1 shows a flow chart of a possible exemplary embodiment of a computer-implemented method according to a first aspect of the present invention.

As can be seen in the flow chart of Fig. 1 the invention provides according to a first aspect a computer-implemented method for sharing data between customer institutions INSTs participating in a digital health platform. The method comprises in a preferred embodiment several main steps S as illustrated in the flow chart of Fig. 1

In a first step S1 at least one data compartment DC adapted to store data of an application of a source institution is provided.

The data compartment DC can comprise in a possible embodiment a discrete storage area which can be established for a specific customer institution. The data compartment DC can serve multiple purposes such as an interim cloud-based storage for a specific customer facing application or as a data collection space for the customer for potential research scenarios.

A customer within the purview of his institution INST can purchase as many applications APPs as he wants and each of these applications APPs might have its own data privacy and security requirements.

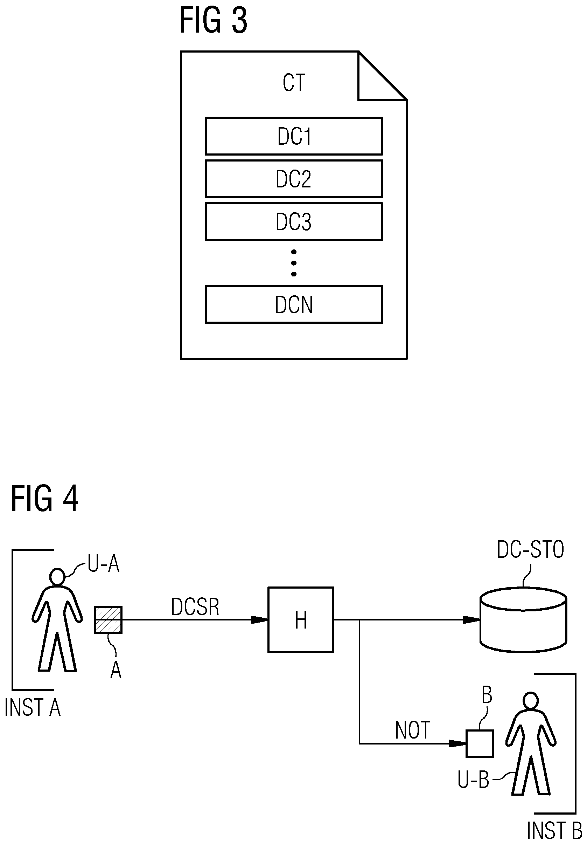

Fig. 3 illustrates a customer tenant CT of an institution INST comprising a number N of data compartments DCs (DC-1 to DC-N) .

In a preferred embodiment the data compartments DCs as illustrated in Fig. 3 are logically isolated from one another and hence the data stored in them can have its own properties. These properties can comprise the actual purpose of the data collection and data minimization privacy profiles applied along with its extensions which are then used to control the level of retained data in the respective data compartment DT. The properties can further comprise tailored data retention policies so that customer can control how long the data can remain in the data compartment DC.

A user or customer can create via a user interface UI of a terminal or user equipment a data compartment DC in the context of his institution INST. The user can trigger the sharing of this data compartment DC. This opens a wide range of possibilities and options for the user so that it supports the user in various workflows.

In a possible embodiment of the method according to the present invention, the data stored in the data compartment DC comprises associated data properties. These data properties can include an indication of a purpose of data stored in the respective data compartment DC. The data properties can also include data retention policies indicating a level of retention and/or a duration of data stored in the respective data compartment DC and a privacy level used for data minimization operation.

In a further step S2 of the method illustrated in Fig. 1 a data compartment DC can be selected by a user of the source institution INST A via a user interface UI of the user's terminal. The terminal can automatically generate a corresponding data compartment sharing request DCSR for sharing the selected data compartment DC with at least one specified target institution INST B.

Fig. 4 illustrates the sharing of a data compartment DC by a user of a source institution INST A.

An authorized user or customer U-A of a source institution (INST A) such as an administrator can select a data compartment DC of the source institution INST A through a user interface UI of his terminal device A.

A target institution (INST B) is specified in a possible embodiment by the user of the source institution INST A by indicating an institution name, an identifier, a serial number and/or an address of the target institution INST B. The data can be input by the user through a user interface UI of the terminal device. One or more target institutions can be selected by the user of the source institution from a displayed list of participating institutions.

In a possible embodiment the generated data compartment sharing request DCSR specifies at least one owner application or at least one third party application available on the target institution INST B to have access to the data stored in the selected data compartment DC of the source institution INST A.

In a possible embodiment of the method according to the present invention the generated data compartment sharing request DCSR further specifies a time frame indicating a time period where the data sharing is active. The data compartment sharing request DCSR can further specify a level of access to the data stored in the selected data compartment DC of the source institution INST A. The level of access specified in the data compartment sharing request DCSR can comprise allowed data operations including reading data, writing data, deleting data and execution of operations on data.

The customer or user U-A belonging to the source institution INST A is enabled by an enabling or authorization mechanism to share an existing or created data compartment DC of an application with another institution referred to as the target institution INST B. While initiating the sharing action the sharing user U-A can be empowered to make sure that the correct target institution INST B is chosen via details such as institution name, country, serial number, address etc. of the target institution INST B.

While initiating the sharing action the user U-A of the source institution INST A can be offered a default selection of the owner application of the data compartment DC if the application is already licensed and available in the target institution INST B.

The user U-A of the source institution INST A can further be empowered to clearly identify a third-party application which is licensed and available on the target institution INST B to have access to the data in the target institution context. The user U-A can also specify a time-frame until the sharing is active (permanent or time limited).

The generated data compartment sharing request DCSR can be sent to a hub H as illustrated in Fig. 4. The hub H can be for instance a DICOM Hub. The share action can be stored in a data compartments storage DC-STO as illustrated in Fig. 4.

In a further step S3 of the computer-implemented method according to the present invention shown in the flow chart of Fig. 1 a user U-B of the target institution INST B is notified about the data compartment sharing request DCSR of the user U-A of the source institution INST A via a user interface UI of its terminal device B as also illustrated in the schematic diagram of Fig. 4. Once the sharing action has been initiated, the customers can be notified about the initiated sharing action via different technical means such as notifications, emails etc.

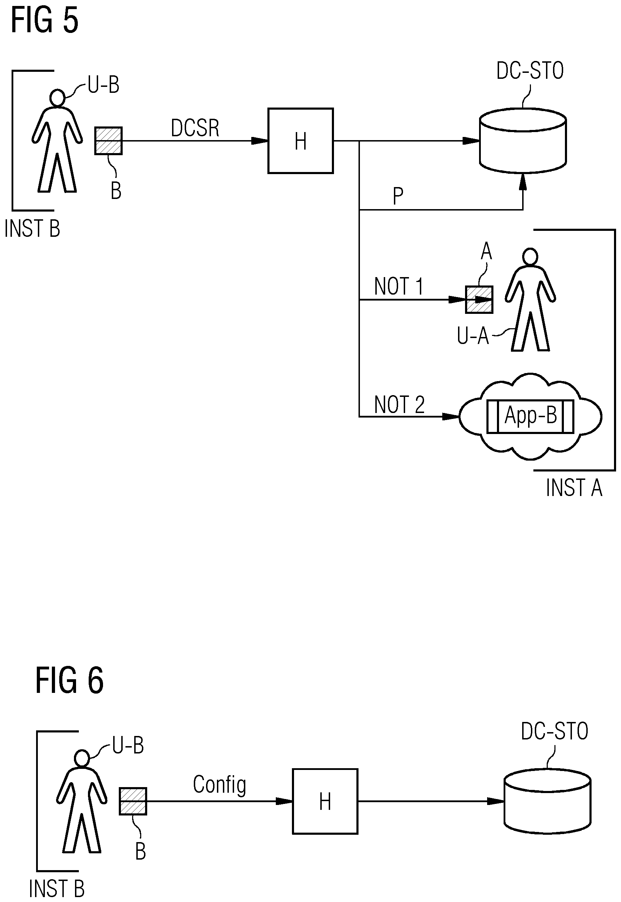

In a further step S4 of the computer-implemented method according to the present invention shown in the flow chart of Fig. 1 the data compartment sharing request DCSR of the user of the source institution INST B is either approved or rejected by a user U-B of the target institution INST B. An authorized customer or user(such as an administrator) U-B belonging to a target institution INST B is enabled to approve or to reject an existing data compartment sharing request DCSR received from another customer belonging to another institution, i.e. a source institution INST A. The approval or rejection of the received data compartment sharing request DCSR can be sent from a terminal device B of the customer of the target institution INST B back to the hub H as illustrated in Fig.5. The hub H can comprise a DICOM hub.

While assessing the received data compartment sharing request DCSR, the user of the target institution INST B is empowered to make sure that he is confirming the correct source institution via details such as institution name, country, serial number, address etc. Further while assessing the received data compartment sharing request DCSR, the user U-B of the target institution INST B can be provided with means to find out the level of access with respect to various operations on data such as create, read, delete, and execute the respective data.

In a further step S5 of the computer-implemented method according to the present invention shown in the flow chart of Fig. 1 the user U-A of the source institution INST A is notified (NOT 1) about the approval or rejection of his data compartment sharing request DCSR by the user U-B of the target institution INST B as also illustrated in Fig. 5.

Once the received data compartment sharing request DCSR has been approved or rejected in step S4 by the user U-B of the target institution INST B, the terminal A of the requesting user or customer of the source institution INST A can be notified in step S5 about the approval/rejection via means such as notifications NOTs , emails etc.as also illustrated in Fig.5.

There can also be notifications (NOT 2) sent to third-party applications (web hook) whenever an existing data compartment DC is shared with them so that they can perform necessary initial setup operations, if necessary, as also illustrated in Fig. 5.

In a further step S6 of the computer-implemented method according to the present invention shown in the flow chart of Fig. 1 access to the data stored in the selected shared data compartment DC of the source institution INST A is provided to the target institution INST B. The access is provided as soon as the terminal device A of the user U-A of the source institution INST A has received an approval notification from the terminal device B of the user U-B of the target institution INST B in response to its original data compartment sharing request DCSR.

In a possible embodiment of the method according to the present invention an authorized user U-B of the target institution INST B is enabled to configure the shared data compartment DC of the source institution INST A using for example a configuration mask displayed on a user interface UI of the terminal device B of the authorized user U-B.

In a possible embodiment, the user U-B can also create policies P for the shared source data compartments DC stored in the data compartment storage DC-STO as illustrated in Fig. 5. This option can be given to the user U-B by the user U-A of the source institution INST A in the data compartment sharing request DCSR.

As illustrated in Fig. 5, affected third party applications APPBs are notified in a notification NOT2 automatically about the data compartments DCs shared with them.

Fig. 6 illustrates the configuration (Config) of shared data compartments DCs. An authorized user U-B of the target institution INST B such as an administrator can view and edit the data compartment configuration via a configuration user interface UI and supply the configuration to a hub H, in particular a DICOM hub. This leads to an update of the configuration of the data compartments DCs stored in the data compartments storage DC-STO as illustrated in Fig.6. Application entity titles (AETs) of application entities AEs can be stored and used for query and retrieval of data.

The invention further provides according to a further aspect a system 1 for sharing data between customer institutions INSTs participating in a digital health platform

As illustrated schematically in the block diagram of Fig. 2, the system 1 comprises a compartment storage 2 and a cloud based distributed system 3.

The data compartment storage (DC-STO) 2 of the system 1 is adapted to store data in logically isolated data compartments DCs established for customer institutions INSTs.

The cloud based distributed system 3 shown in Fig. 2 is adapted to receive a data compartment sharing request DCSR for sharing a selected data compartment DC with a specified target institution INST B in response to a selection of a data compartment DC of a source institution INST A by a user U-A of the source institution INST A. The cloud based system 3 is further adapted to provide automatically access to the data stored in the selected data compartment DC of the source institution INST A to a user or customer U-B of the target institution INST B if the data compartment sharing request DCSR has been approved by an authorized user U-B of the target institution INST B.

The cloud based distributed system 3 is further adapted to notify a terminal device B of the user U-B of the target institution INST B about the received data compartment sharing request DCSR issued by the terminal device A of the user U-A of the source institution INST A. The cloud based distributed system 3 can further notify the user U-A of the source institution INST A about an approval or rejection of his data compartment sharing request DCSR according to the processed decision of the user U-B of the target institution INST B.

In a further possible embodiment of the cloud based distributed system 1 the system further comprises a network connecting a plurality of distributed customer institutions INSTs and adapted to transport messages and notifications between the institutions INSTs according to a predefined communication protocol.

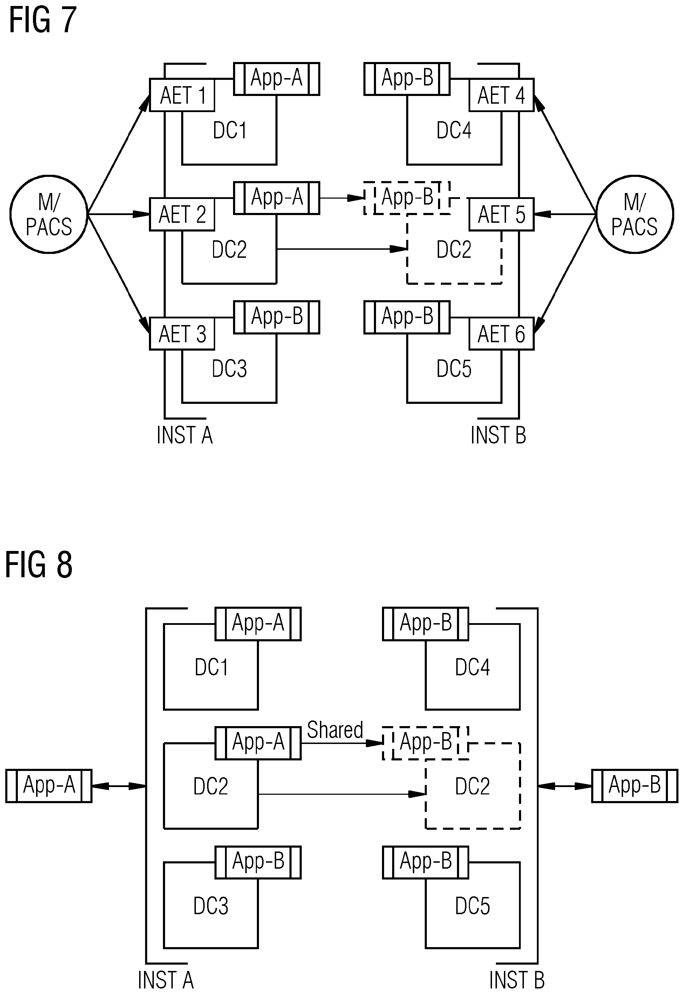

In a still further possible embodiment of the cloud based distributed system 1 the customer institutions INSTs form application entities AEs in a DICOM (Digital Imaging and Communications in Medicine) network, wherein each data compartment DC exposes unique application entity titles (AETs) as also illustrated in Fig. 7.

In the illustrated example of Fig. 7, the source institution INST A comprises a storage with three data compartments DC1, DC2, DC3. In the illustrated example of Fig. 7, the second data compartment DC 2 of the source institution INST A is shared with the target institution INST B by performing the computer-implemented method according to the present invention. A modality M or PACS of the institutions INSTs A, B has access to the data compartments DCs of the respective institution INST for further processing of data stored therein.

DICOM is an international standard for medical images and related information. It defines a medical image format that can be used for data exchange with a quality that meets clinical needs. DICOM is used in radiology, cardiovascular imaging and radiological diagnosis and treatment equipment (such as X-ray equipment, CT equipment, magnetic resonance equipment, ultrasound equipment, etc.), and has been widely used in medical fields such as ophthalmology and dentistry.

DICOM files not only contain information about the image itself, but also carry medical-related information. Using the DICOM standard simplifies the realization of medical image information exchange, and promotes the research and development of Image Archiving and Communication Systems (PACS).The DICOM files contain both image data and metadata. The image data represents the actual medical images, such as X-rays, CT scans, MRIs, and more. The metadata includes information about the patient, imaging equipment, acquisition parameters, and other details related to the image.

The DICOM files have a specific structure, including a header and image data. The header contains information in a tag-value format and is organized in Data Elements. Each Data Element has a unique tag, and the data can include patient demographics, study information, image acquisition details, and more.

DICOM uses a specific communication protocol to exchange information between different devices and systems. The most common transport protocols used with DICOM are TCP/IP (Transmission Control Protocol/Internet Protocol). This allows for the secure and reliable transfer of medical images and associated data over networks.

DICOM defines various service classes that describe different aspects of medical imaging communication. Examples include the Storage Service Class for storing and retrieving images, the Query/Retrieve Service Class for querying and retrieving information from a PACS (Picture Archiving and Communication System), and the Modality Worklist Service Class for managing worklists for imaging devices.

In a possible embodiment of the cloud based distributed system the system comprises a DICOM server comprising for its identification and communication an IP-number, a port number and one or more application entity titles (AETs) belonging to a single or multiple data compartments.

An Application Entity (AE) is a node in a DICOM network, which can be any device or software application that communicates using the DICOM standard. Each Application Entity is identified by a unique Application Entity Title (AET), which serves as a label or identifier for that entity in the DICOM network.

The Application Entity Title AET is used during the DICOM communication process to specify the source and destination of DICOM messages. When devices or applications communicate over a DICOM network, they include their Application Entity Title AET in the DICOM communication protocol to identify themselves. This ensures that the correct entities are sending and receiving DICOM data.

For example, in a DICOM network, a Picture Archiving and Communication System (PACS), a radiology information system (RIS), or a medical imaging device (such as a CT scanner or an MRI machine) can have a unique AET. When one entity wants to communicate with another, it uses the AET of the target entity in the DICOM communication to address the messages correctly.

A customer can create or edit DICOM AE title(s) to send DICOM data via a network to the shared data compartment DC. The customer can further create or edit DICOM AE title(s) to query and retrieve DICOM data via a network from the shared data compartment DC. Uniqueness of the AETs is ensured to avoid duplicates within the tenant.

A customer/user belonging to an institution INST is enabled to configure a data compartment DC which was previously shared with the respective institution INST. It is possible to differentiate the shared data compartment from regular data compartments of the institution INST and to access them in a similar fashion.

The system 1 provides the ability to have a read-only look at the properties of the data compartment DC and associated owner application APP. The system 1 can further provide the user with the ability to have a read-only look at the provided policies related to various data types such as DICOM, attachments and re-identification.

DICOM provides a standardized way for different medical imaging devices and systems to communicate, ensuring interoperability and the efficient exchange of medical image data in healthcare environments. This standard provides accessibility and usability of medical images across various healthcare settings.

In the method and system according to the present invention, data compartments DC can be shared with one or more third party applications APPs during the sharing action. There can be mechanisms for the third-party applications to get to know about the data compartments DCs which are shared with them as also illustrated in Fig. 8.

In a possible embodiment, a public API provides detailed information about the shared / available data compartments DC for a given application APP in the context of a specific institution INST. Existing public APIs around QIDO-RS, WADO-RS, STOW-RS are adapted to respect the policies added as part of the data compartment sharing. They can grant access to the data accordingly to the respective application APP in the context of the shared institution.

As shown in the example of Fig. 8, an application APP can upon request get be informed about data compartments DCs owned by the respective application APP at the local institution INST and about data compartments DCs shared with the respective APP from another institution INST. For instance application APP A can request to get all data compartments DCs owned by it for institution A which returns that data compartments DC1 and data compartment DC2 of institution A are owned by application APP A. Further application B can request to get all data compartments DCs owned by it for institution B which returns that data compartments DC4 and data compartment DC5 of institution A are owned by application APP B and that data compartment DC2 is shared with application B from institution A as illustrated in Fig. 8.

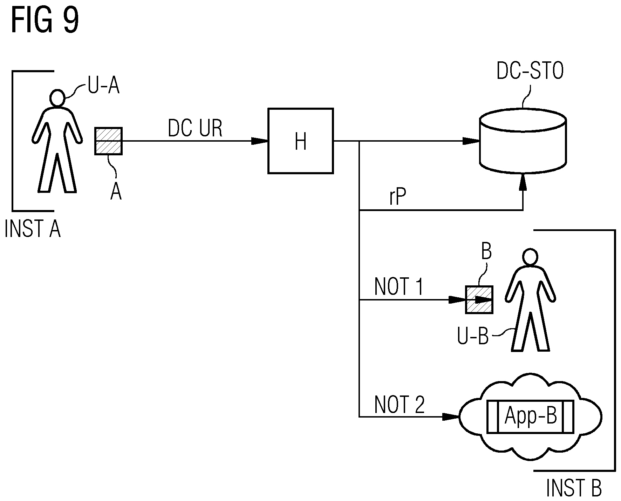

In a possible embodiment, a customer or user belonging to an institution INST is enabled to un-share an existing data compartment DC of an application APP with another institution as illustrated in Fig. 9. A customer belonging to an institution INST can be enabled to automatically un-share (auto expiry) an existing data compartment DC of an application based on the initially configured time-frame.

In a possible embodiment as illustrated in Fig. 9, sharing of the shared data compartment DC of the source institution INST A can be terminated in response to a data compartment un-sharing request DCUR triggered by a user U-A of the source institution INST A sent to a hub H. The data compartment un-sharing request DCUR can also be triggered by an event, in particular by the expiration of the time-period where the data sharing is active as specified in the data compartment sharing request DCSR.

In a possible embodiment, a terminal device B of a user U-B of the target institution INST B and affected third party applications APPBs are notified by notification NOT1, NOT2 automatically about a termination of the sharing of the data within the data compartment DC of the source institution INST A as shown in Fig. 9.

A removal of the share (manual or automatic) can have different consequences. The removal of the share can lead to a removal of existing data ingestion channels such as a removal of configured DICOM AE titles from the target institution INST B. Further the policies of the data compartment DC for the target institution and the application are removed (rP). The affected third-party application APP is notified about the removal of the compartment share as well (NOT 2).

In a possible embodiment of the cloud based distributed system 1, the system 1 is inter-operable with one or more other connected systems (not shown in Fig. 2).

These other systems can include imaging modalities, in particular medical imaging devices including X-ray machines, CT scanners, MRI machines adapted to generate medical images.

The other systems can also include modality worklist servers adapted to manage worklists for imaging modalities and/or providing information about scheduled procedures.

The other systems can further include image acquisition devices for acquiring medical images from imaging modalities and sending them to a Picture Archiving and Communication System (PACS) adapted to store, retrieve, and distribute medical images.

The other systems can further include viewer components adapted to retrieve medical images from the Picture Archiving and Communication System (PACS) and used by healthcare professionals to view and interpret the retrieved medical images.

The other systems can further include Radiology Information Systems (RIS) adapted to manage radiological information, including patient scheduling, reporting, and other administrative functions.

The other systems can further include print servers used for printing medical images to hardcopy films or other output devices.

A possible use case of the method and system according to the present invention is to provide a network of institutions INSTs coming together and working towards offering a stroke expert network and to enable easy diagnosis of patients with a stroke irrespective of where they are located. Each participating institution INST can create its own data compartments DCs. Each of the participating institutions INST can then share the whole data compartment DC with another institution where the stroke experts are residing. The provision of these shared data compartments DCS enables access to data via various means such as via cloud data APIs, via automatic data transfer to their local systems through DICOM TCP/IP, via cloud-based user interfaces.

A further exemplary use case of the method and system according to the present invention is to offer radiology services to hospitals across the world. The concept of this use case is similar to the first use case except that in this situation, there can be multiple Artificial Intelligence(AI) applications including external ones which can be connected to the data compartments DCs. Data ingestion can be provided from all involved institutions INSTs in both directions.

A further exemplary use case of the method and system according to the present invention is the provision of hospitals offering tele radiology for customers in a specific geographic region making use of cross institution data compartments.

The digital health platform DHP employing the method according to of the present invention can connect user/customers, institutions INSTs and applications APPs with each other via a secure data sharing and connectivity service. The platform is vendor-, device- and system-neutral to enable standardization and to support seamless interoperability across departments and institutions. It enables a cross-institutional exchange of electronic health records EHRs or other data and thus supports collaboration between care teams and connects them to patients to facilitate successful cooperation.

The different embodiments described above may be combined with each other.

Although the present invention has been described above on the basis of preferred embodiments it is not restricted to these, but can be modified in different ways. For instance the notifications, messages and shared data can be transmitted in encrypted form. Further only authorized users may be allowed to install sharing data compartments of an institution with other institutions. Further, the data sharing can also be initiated by a user U-B of a target institution INST B seeking access to data of a source institution INST A. In this variant the user U-B may sent or broadcast an inquiry to one or more source institutions INST A to get access to specific data compartments DCs. The address user U-A can then create data compartment sharing request DCSR if they accept the inquiry.

Although the present invention has been described in the above by way of embodiments, it is not limited thereto, but rather can be modified in a wide range of ways. In particular, the invention can be changed or modified in various ways without deviating from the core of the invention.

Independent of the grammatical usage, individuals with male, female or other gender identities are included within the term.

## Claims

1. A computer-implemented method for sharing data between customer institutions (INST) participating in a digital health platform, the method comprising:
providing (S1) at least one data compartment (DC) adapted to store data of an application (APP) of a source institution (INST A) ;
receiving (S2) a data compartment sharing request (DCSR) issued by a user (U-A) of the source institution (INST A) via a terminal device (A) of the respective user (U-A), wherein the data compartment sharing request (DCSR) is a request for sharing a data compartment (DC) selected by the user (U-A) with a specified target institution (INST B);
notifying (S3) a terminal device (B) of a user (U-B) of the target institution (INST B) about the data compartment sharing request (DCSR);
receiving (S4) an approval or rejection of the data compartment sharing request (DCSR) issued by the user (U-B) of the target institution (INST B) via the terminal device (B) of the user (U-B) of the target institution (INST B);
notifying (S5) the terminal device (A) of the user (U-A) of the source institution (INST A) about the approval or rejection of the data compartment sharing request (DCSR); and
in the case of an approval, providing (S6) access to the data stored in the selected data compartment (DC) of the source institution (INST A) to the terminal device (B) of a user (U-B) of the target institution (INST B).

2. The method according to claim 1, wherein the data compartment sharing request (DCSR) issued by the user (U-A) of the source institution (INST A) comprises an institution name, an identifier, a serial number and/or an address of the target institution (INST B).

3. The method according to any of the preceding claims, wherein the data compartment sharing request (DCSR) specifies at least one owner application or at least one third party application available on the target institution (INST B) to have access to the data stored in the selected data compartment (DC) of the source institution (INST A).

4. The method according to any of the preceding claims, wherein the data compartment sharing request (DCSR) specifies a time frame indicating a time period where the data sharing is active.

5. The method according to any of the preceding claims, wherein the data compartment sharing request (DCSR) specifies a level of access to the data stored in the selected data compartment (DC) of the source institution (INST A).

6. The method according to claim 5, wherein the level of access specified in the data compartment sharing request (DCSR) comprises allowed data operations including reading data, writing data, deleting data and execution of operations on data.

7. The method according to any of the preceding claims, wherein the data stored in the data compartment (DC) comprises associated data properties including an indication of a purpose of data stored in the respective data compartment (DC) and data retention policies indicating a level of retention and/or a duration of data stored in the respective data compartment (DC) and a privacy level used for data minimization operation.

8. The method according to any of the preceding claims, wherein the user (U-B) of the target institution (INST B) is enabled by an authorization mechanism to configure the shared data compartment (DC) of the source institution (INST A).

9. The method according to claim 3, wherein the affected third party applications are notified automatically about the data compartments (DCs) shared with them.

10. The method according to any of the preceding claims, comprising receiving a data compartment un-sharing request (DCUR) triggered by a user of the source institution (INST A) or triggered by an event, in particular by the expiration of the time period where the data sharing is active as specified in the data compartment sharing request (DCSR), and terminating sharing of the shared data compartment (DC) of the source institution (INST A) in response to the received data compartment un-sharing request (DCUR).

11. The method according to claim 10, wherein the user (U-B) of the target institution (INST B) is notified automatically about a termination of the sharing of the data compartment (DC) of the source institution (INST A).

12. A system (1) for sharing data between customer institutions participating in a digital health platform, the system (1) comprising:
a compartment storage (2) adapted to store data in logically isolated data compartments (DCs) established for customer institutions(INSTs); and
a cloud based distributed system (3) adapted to receive a data compartment sharing request (DCSR) issued by a user (U-A) of a source institution (INST A) via a terminal device (A) of the respective user (U-A), wherein a data compartment sharing request (DCSR) is a request for sharing a selected data compartment (DC) with a specified target institution (INST B) and adapted to provide automatically access to the data stored in the selected data compartment (DC) of the source institution (INST A) to the target institution (INST B) if an approval of the data compartment sharing request (DCSR) is received from a terminal device (B) of a user (U-B) of the target institution (INST B).

13. The system according to claim 12, wherein the cloud based distributed system (3) is further adapted to notify a user (U-B) of the target institution (INST B) about the received data compartment sharing request (DCSR) of the user (U-A) of the source institution (INST A) and to notify the user (U-A) of the source institution (INST A) about the approval or rejection of the data compartment sharing request (DCSR) by the user (U-B) of the target institution (INST B).

14. The system according to claim 12 or 13, further comprising a network connecting a plurality of customer institutions (INSTs) and adapted to transport messages and notifications between the institutions (INSTs) according to a predefined communication protocol.

15. The system according to claim 14, wherein the customer institutions (INSTs) form application entities (AEs) in a DICOM (Digital Imaging and Communications in Medicine) network, wherein each data compartment (DC) exposes unique application entity titles (AETs).

16. The system according to any of the preceding claims 12 to 15, wherein the cloud based distributed system (2) comprises a DICOM server comprising for its identification and communication an IP-number, a port number and one or more application entity titles (AETs) belonging to a single data compartment (DC) or multiple data compartments (DC).

17. The system according to any of the preceding system-based claims 12 to 16, wherein the system (1) is inter-operable with other systems including:
imaging modalities, in particular medical imaging devices including X-ray machines, CT scanners, MRI machines adapted to generate medical images;
modality worklist servers adapted to manage worklists for imaging modalities and/or providing information about scheduled procedures;
image acquisition devices for acquiring medical images from imaging modalities and sending them to a Picture Archiving and Communication System (PACS) adapted to store, retrieve, and distribute medical images;
viewer components adapted to retrieve medical images from the Picture Archiving and Communication System (PACS) and used by healthcare professionals to view and interpret the retrieved medical images;
Radiology Information Systems (RIS) adapted to manage radiological information, including patient scheduling, reporting, and other administrative functions;
print servers used for printing medical images to hardcopy films or other output devices.

18. A computer program comprising instructions which, when the program is executed by a computer, cause the computer to carry out the steps of the method of one of the claims 1 to 11.

19. A computer-readable medium comprising instructions which, when executed by a computer, cause the computer to carry out the steps of the method of one of the claims 1 to 11.
